Europäisches Patentamt

European Patent Office

Office européen des brevets

(11) Veröffentlichungsnummer: **0 269 831**
**A2**

(12) **EUROPÄISCHE PATENTANMELDUNG**

(21) Anmeldenummer: 87115491.0

(51) Int. Cl.⁴: **A61K 7/15**

(22) Anmeldetag: 22.10.87

(30) Priorität: 12.11.86 DE 3638670

(43) Veröffentlichungstag der Anmeldung:
08.06.88 Patentblatt 88/23

(84) Benannte Vertragsstaaten:
AT BE CH DE ES FR GB GR IT LI NL SE

(71) Anmelder: **Beiersdorf Aktiengesellschaft**
**Unnastrasse 48**
**D-2000 Hamburg 20(DE)**

(72) Erfinder: **Borkowski, Norbert, Dr.**
**Senzoku Compound B 1-4-19 Kitasenzoku**
**Ohta-ku Tokyo(JP)**
Erfinder: **Krause, Karl-Heinz**
**Tucheler Weg 1d**
**D-2000 Norderstedt 1(DE)**
Erfinder: **Heinig, Regine**
**Hagedornstrasse 47**
**D-2000 Hamburg 13(DE)**
Erfinder: **Schmidt-Lewerkühne, Hartmut, Dr.**
**Dornkamp 14**
**D-2000 Schenefeld(DE)**

(54) Nachschäumendes kosmetisches Gel.

(57) Nachschäumendes kosmetisches Gel, insbesondere ein Rasiergel auf der Basis eines wässrigen Seifengels zur Abgabe aus einem Aerosolbehälter, das mindestens einen Celluloseether und/oder mindestens ein Acrylsäurepolymerisat und mindestens einem Zuckeralkohol als Gelstrukturbildner sowie ausschließlich Chlorfluorkohlenwasserstoffe als Nachschäummittel enthält.

EP 0 269 831 A2

## Nachschäumendes kosmetisches Gel

Die Erfindung betrifft ein nachschäumendes kosmetisches Gel, insbesondere ein Rasiergel auf der Basis eines wässrigen Seifengels zur Abgabe aus einem Aerosolbehälter, das mindestens einen Celluloseether und/oder mindestens ein Acrylsäurepolymerisat und mindestens einem Zuckeralkohol als Gelstrukturbildner sowie ausschließlich Chlorfluorkohlenwasserstoffe als Nachschäummittel enthält.

Derartige nachschäumende kosmetische Gele werden bei Anwendung zunächst mit Hilfe eines Ausbringmittels gelförmig aus dem Aerosolbehälter auf die Haut aufgebracht und entwickeln erst dort nach kurzer Verzögerung unter dem Einfluß des enthaltenen Nachschäummittels den eigentlichen Schaum. Der Vorteil dieser Zusammensetzungen gegenüber den bekannten fertigen kosmetischen Schäumen, die bereits geschäumt aus dem Aerosolbehälter auf die Haut aufgebracht werden, liegt in einer besseren Benetzung der Haut bzw. der Barthaare. Dies bewirkt insbesondere bei nachschäumenden Rasiergelen eine vollständigere Befeuchtung des Bartes und ein besseres Gleiten des Rasierapparates.

Nachschäumende kosmetische Gele sind vor allem in Form von Rasiergel-Zusammensetzungen im Prinzip bekannt. Die US-PS 3,541, nennt als essentielle Bestandteile einer solchen Zusammensetzung Wasser, Seife (d.h. wasserlösliche Salzehöherer Fettsäuren), Gelstrukturbildner und Nachschäummittel. Zweckmäßig, jedoch nicht unbedingt erforderlich ist darüberhinaus der Zusatz von kosmetischen Wirk-und Hilfsstoffen. Es ist auch vorgeschlagen worden (US-PS 4,405,489), auf einen Gelstrukturbildner zu verzichten, jedoch ist in diesem Falle ein spezieller und aufwendiger Prozeß für die Herstellung und Abfüllung derartiger Gel-Zusammensetzungen erforderlich.

Im einzelnen enthält die nachschäumende Gel-Zusammensetzung nach der obenerwähnten US-PS 3,541,581 40-90 Gew.-% Wasser, 4-25 Gew.-% wasserlösliche Seife, 0,5-12 Gew.-% Nachschäummittel und 0,01-5 Gew.-% Gelbildner. Als Gelbildner finden dabei wasserlösliche Derivate von Cellulose, Sucrose und Glucose Verwendung, insbesondere Copolymere von Acrylsäure und Polyallylsucrose oder die Reaktionsprodukte von Cellulose oder Glucose mit Säuren oder Alkylenoxiden.

Um die Fließeigenschaften bei der Abgabe aus dem Aerosolbehälter zu verbessern, schlägt die DE-OS 24 13 122 vor, einen Wasseranteil im oberen Bereich (75-90 Gew.-%) und einen Seifenanteil im unteren Bereich (5-15 Gew.-%, vorzugsweise 5-9 Gew.-%) der vorgenannten Zusammensetzung zu wählen.

Die beschriebenen nachschäumenden kosmetischen Gel-Zusammensetzungen weisen jedoch insbesondere bei der Herstellung entscheidende Nachteile auf: Als Nachschäummittel werden aliphatische Kohlenwasserstoffe verwendet, vorzugsweise n-Butan, Pentane und Hexane. Diese Verbindungen sind brennbar und bilden mit Luft explosionsfähige Gemische. Daher ist bei der Fertigung ein erhöhter Aufwand für Explosions-Schutz und ähnliche Sicherheitsmaßnahmen erforderlich. Zwar sind in der US-PS 3,541,581 auch Fluorchlorethane als mögliche Nachschäummittel genannt, welche nicht brennbar sind, jedoch haben diese in der Praxis keine Bedeutung erlangen können. Auch die US-PS 3,541,581 bietet keine Rezepturbeispiele für nachschäumende Rasiergel-Zusammensetzungen mit Fluorchlorethanen als Nachschäummittel.

Aufgabe der vorliegenden Erfindung war es daher, eine den Anforderungen der Praxis entsprechende nachschäumende kosmetische Gel-Zusammensetzung, insbesondere ein Rasiergel nach Art der bekannten, einen Gelbildner enthaltenden wässrigen Seifengele zu schaffen, die als Nachschäummittel eine nicht brennbare, im Luftgemisch nicht explosionsfähige Verbindung enthält und damit eine problemlosere und zugleich auck kostengünstigere Herstellung ermöglicht.

Aufgabe der Erfindung war es weiterhin, auf der Basis der erfindungsgemäßen nachschäumenden kosmetischen Gel-Zusammensetzung weitere nachschäumende kosmetische Produkte zur Verfügung zu stellen, beispielsweise ein nachschäumendes Gel zur Hautpflege oder zur Hautreinigung.

Diese Aufgabe wurde überraschenderweise durch den Einsatz eines speziellen neuen Gelstrukturbildner-Gemisches sowie daran angepaßte veränderte Einsatzmengen der übrigen Komponenten gelöst.

Gegenstand der Erfindung ist daher ein nachschäumendes kosmetisches Gel, insbesondere ein Rasiergel, auf der Basis eines wässrigen Seifengels zur Abgabe aus einem Aerosolbehälter, dadurch gekennzeichnet, daß es neben Wasser, Fettsäuresalz (Seife) und üblichen kosmetischen Wirk-und Hilfsstoffen als Gelstrukturbildner ein Gemisch aus 0,05 - 4 Gew.-%, vorzugsweise 0,1 - 1,0 Gew.-%, mindestens eines Celluloseethers und/oder mindestens eines Acrylsäurepolymerisats und 10 - 20 Gew.-%, vorzugsweise 10 - 15 Gew.-% mindestens eines Zuckeralkohols, sowie als Nachschäummittel ein Gemisch aus 8 -20 Gew.-%, vorzugsweise 8 - 15 Gew.-% mindestens eines unter Normalbedingungen (20°C / 1013 mbar) flüssigen Fluorchlorkohlenwasserstoffen als Lösungsmittelkomponente und 4 -10 Gew.-% mindestens eines unter Normalbedingungen gasförmigen Fluorchlorkohlenwasserstoffes als Treibgaskomponente enthält.

Als Celluloseether werden erfindungsgemäß bevorzugt Carboxymethylcellulose, Methyl-und Ethylcellu-

2

lose sowie Hydroxyalkylcellulose mit 1 - 4 C-Atomen in der Alkylkette und Gemische dieser Verbindungen eingesetzt, insbesondere wird Hydroxypropylcellulose verwendet.

Gemäß der Erfindung bevorzugte Acrylsäurepolymerisate sind solche, die Acrylsäureesteranteile enthalten. Derartige Verbindungen sind beispielsweise unter dem Handelsnamen Carbopol 1342® (B.F. Goodrich Corp., Cleveland) auf dem Markt.

Als Zuckeralkohole für ein nachschäumendes kosmetisches Gel, insbesondere ein Rasiergel gemäß der Erfindung, eignen sichvorzugsweise Polyalkohole mit 3 -6 C-Atomen in der Kette, in der besonders bevorzugten Ausführungsform wird Sorbit eingesetzt.

Als Fluorchlorkohlenwasserstoffe, die gemäß der Erfindung als Lösungsmittelkomponented es Nachschäummittels dienen können,eignen sich wasserstoff-freie Ethan-Derivate, die nur Fluor-und Chlorsubstituenten tragen, mit einem Siedepunkt zwischen 20 und 50°C und deren Gemische. Gemäß der bevorzugten Ausführungsform der Erfindung wird 1,1,2-Trichlor-1,2,2,trifluorethan eingesetzt.

Als erfindungsgemäße Treibgaskomponente des Nachschäummittels eignen sich perhalogenierte, nur Fluor-und Chlorsubstituenten tragende Methan-und Ethan-Derivate, bevorzugt Gemische aus mindestens 2 dieser Verbindungen, besonders bevorzugt Gemische aus einem Methan-und einem Ethan-Derviat, insbesondere Gemische aus Dichlordifluormethan und 1,2-Dichlortetrafluorethan, vorzugsweise in einem Verhältnis von 1: 2,6 bis 1 : 16, insbesondere 1 : 2,6 bis 1 : 10.

Es ist vorteilhaft, den Wassergehalt der erfindungsgemäßen nachschäumenden kosmetischen Gel-Zusammensetzung auf maximal 60 Gew.-% zu beschränken, bevorzugt ist ein Wassergehalt von 45 bis 60 Gew.-%.

Der Gehalt an Fettsäuresalz (Seife) beträgt erfindungsgemäß vorzugsweise 10-20 Gew.-%. Bei den Fettsäuresalzen handelt es sich um kosmetisch akzeptable, wasserlösliche Salze von gesättigten aliphatischen Carbonsäuren mit 12-18, vorzugsweise 14-18 C-Atomen, vorzugsweise um die Kalium-, Ammonium-und löslichen Aminsalze. Geeignete Verbindungen sind dem Fachmann beispielsweise aus der bereits zitierten DE-OS 24 13 122 bekannt. Besonders bevorzugt sind für die Ausführung der Erfindung di Triethanolaminsalze der Stearin-, Palmitin-oder Myristinsäure oder Gemische dieser Salze.

Der erfindungsgemäßen nachschäumenden Rasiergel-Zusammensetzung werden bevouzgt zususätzlich 0,5 - 6,5 Gew.-% eines oder mehrerer Verdicker zugefügt, beispielsweise Alkanolamide und deren Derivate. Diese Verbindungen könne teilweise zusätzlich einen hautpflegenden Effekt haben. Erfindungsgemäß besonder bevorzugt ist der Einsatz von 0,5 - 3,0 Gew.-% Fettsäurediethanolamid,wobei besonders das Diethanolamid der Kokosfettsäure bevorzugt wird, und 0,5 - 3,5 Gew.-% Laureth-2-Amid-MEA (Aminol®A15 der Firma Chem-Y) jeweils allein oder als Gemisch.

Weiterhin kann die erfindungsgemäße nachschäumende Rasiergel-Zusammensetzung zusätzlich kleine Mengen üblicher kosmetischer Zusatz-und Hilfsstoffe, z.B. Parfum, Konservierungsmittel, oder Farbstoffe sowie hautpflegende Substanzen wie beispielsweise Proteine, insbesondere Sericin, in Mengen bis zu 0,2 Gew.-% enthalten. Diese Zusätze sind jedoch für die Ausführung der Erfindung nicht unbedingt erforderlich.

Eine weitere Ausführungsform der Erfindung betrifft ein nachschäumendes kosmetisches Gel zur Reinigung und/oder Pflege der Haut auf der Basis eines wässrigen Seifengels zur Abgabe aus einem Aerosolbehälter, das in analoger Weise aus den vorstehend beschriebenen Bestandteilen besteht. Ein solches Gel enthält vorzugsweise zusätzlich mit der Gel-Rezeptur verträgliche, übliche hautpflegende oder hautreinigende Substanzen wie beispielsweise Fettsäureester oder Kollagenderivate.

Die Herstellung des erfindungsgemäßen nachschäumenden kosmetischen Gels erfolgt in einem heiz-bzw. kühlbaren Kessel mit Rührwerk. Dieser Mischapparat muß druck-und vakuumfest sein, insbesondere sollte er ein Arbeiten bei Drucken von mindestens 2 bar zulassen.

Der Seifenkörper wird bei erhöhter Temperatur, vorzugsweise bei 80 bis 90°C gebildet, indem die geschmolzene Fettsäure der wäßrigen Lösung des Verseifungsmittels und des Zuckeralkohols zugefügt wird. Hierbei ist die Rührgeschwindigkeit ebenso wie im nachfolgenden weiteren Prozeß auf die Viskosität des Prozeßgutes derartig abzustimmen, daß stets eine schonende aber ausreichende Durchmischung gewährleistet ist. Die für den Mischprozeß nötigen Rührerdrehzahlen hängen, wie dem Fachmann bekannt ist, von der Art des verwendeten Kessels und insbesondere von der Art des verwendeten Rührwerks ab. Im Falle eines Ankerrührwerks haben sich beispielsweise Drehzahlen von vorzugsweise 10 bis 300 Upm, insbesondere 20 - 40 Upm als geeignet erwiesen.

Nach der Bildung des Seifenkörpers läßt man auf Raumtemperatur abkühlen und setzt die übrigen Bestandteile während der Abkühlphase vorzugsweise in der folgenden Reihenfolge zu:

-Verdicker
-weitere Gelbildner-Komponente(n)
-Farbstoffe

-Parfum

-ggf. Wirkstoffe

-Lösungsmittelkomponente des Nachschäummittels

-Treibgaskomponente des Nachschäummittels.

Die Abfüllung in die innere Kammer eines üblichen Zweikammer-Aerosolbehälters mit einer zweiten, äußeren Kammer für das Ausbringmittel erfolgt anschließend entweder bei Temperaturen unter Raumtemperatur (d.h. weniger als 20°C) oder in einem geschlossenen Drucksystem.

In einer anderen Ausführungsform wird der Mischvorgang vor Zugabe der Treibgaskomponente abgebrochen und die Treibgaskomponente erst während des Abfüllprozesses dem Restgel zugefügt.

In einer weiteren Ausführungsform wird der Mischprozeß bereits vor Zugabe der Lösungsmittelkomponente beendet und Lösungsmittelkomponente und Treibgaskomponente getrennt oder gemeinsam während des Abfüllens zugegeben.

Als Ausbringmittel lassen sich alle für diesen Zweck üblichen und dem Fachmann bekannten komprimierten Gase und Gasgemische einsetzen. Um jedoch auch in diesem Schritt den Vorteil der vergleichsweise problemlosen Verarbeitung zu erhalten, ist es vorteilhaft, für das erfindungsgemäße nachschäumende kosmetische Gel ein nichtbrennbares Gas oder Gasgemisch als Ausbringmittel einzusetzen. Erfindungsgemäß bevorzugt ist deshalb die Verwendung von Stickstoff oder Fluorchlorkohlenwasserstoffen.

Durch die vorstehend beschriebene Erfindung wird eine nachschäumende kosmetische Gel-Zusammensetzung, insbesondere eine Rasiergel, zur Verfügung gestellt, die durch den Einsatz eines neuartigen Gelstrukturbildner-Gemisches zu einem Produkt führt, welchebei einer guten Gleitfähigkeit des Rasierapparates im Vergleich zu bisher bekannten Produkten bezüglich des hautpflegenden Effektes und des Hautgefühls nach der Rasur von den Verwendern besser beurteilt wurde. Darüberhinaus bietet die erfindungsgemäße Rasiergel-Zussammensetzung den großen Vorteil, daß sie kein brennbares und im Luftgemisch explosives Nachschäummittel enthält und damit eine einfachere, sichere und kostengünstigere Produktion ermöglicht.

Die folgenden Beispiele sollen die Erfindung erläutern, ohne sie darauf zu beschränken.

Beispiele 1 - 4

Die folgenden, in Tabelle 1 angegebenen Zusammensetzungen wurden hergestellt und getestet. Alle lieferten nachschäumende Rasiergele mit einwandfreien Gebrauchseigenschaften.

Tabelle 1:

| Bestandteile: (Angaben in Gew.-%) | Beispiel Nr. | | | |
|---|---|---|---|---|
| | 1 | 2 | 3 | 4 |
| Myristinsäure | 1,3 | - | 3,0 | - |
| Palmitinsäure | 1,8 | 4,1 | 4,7 | 1,3 |
| Stearinsäure | 6,9 | 5,4 | 5,8 | 5,7 |
| Polyoxyethylenfettalkoholether (Brij[R]52) | 2,1 | - | - | - |
| Kokosfettsäurediethanolamid | - | 2,8 | - | 1,2 |
| Laureth-2-Amid MEA (Aminol[R]A15) | 3,1 | - | 1,8 | 2,5 |
| Wasser demineralisiert | 49,17 | 48,89 | 49,54 | 50,54 |
| Sorbit | 12,6 | 13,1 | 13,6 | 14,8 |
| Triethanolamin | 6,4 | 6,6 | 5,8 | 7,2 |
| Hydroxypropylcellulose | 0,06 | - | 0,13 | 0,07 |
| Acrylsäure-Polymerisat (Carbopol[R] 1342) | 0,04 | 0,11 | - | 0,05 |
| Sericin | 0,03 | - | 0,03 | 0,04 |
| Farbe | 0,5 | 0,5 | 0,5 | 0,5 |
| Parfum | 0,5 | 0,5 | 0,5 | 0,5 |
| 1,1,2-Trichlor-1,2,2-trifluorethan | 9,2 | 12,3 | 9,0 | 8,4 |
| Dichlordifluorethan/1,2-Dichlortetrafluorethan Verhältnis 1 : 9 | 6,3 | 5,7 | 5,6 | 7,2 |
| | 100,0 | 100,0 | 100,0 | 100,0 |

Die Herstellung wurde in einem heiz-und kühlbaren, druckfesten Kessel mit Ankerrührwerk vorgenommen.

Der Seifenkörper wurde bei 85°C gebildet, indem die geschmolzenen Fettsäuren der wässrigen Lösung aus Triethanolamin und Zuckeralkohol bei einer Rührgeschwindigkeit von 40 Upm zugefügt wurde. Anschließend läßt man abkühlen und gibt während der Abkühlphase unter weiterem Rühren die übrigen Komponenten in folgender Reihenfolge zu:

Verdicker
weitere Gelstrukturbildner-Bestandteile

Farbstoff
Parfum
Wirkstoff (Sericin)
1,1,2-Trichlor-1,2,2-trifluorethan
Dichlordifluorethan/1,2-Dichlortetrafluorethan-Gemisch
Anschließend wird bei 4°C in den Aerosolbehälter abgefüllt.

**Ansprüche**

1. Nachschäumendes kosmetisches Gel auf der Basis eines wässrigen Seifengels zur Abgabe aus einem Aerosolbehälter, dadurch gekennzeichnet, daß es neben Wasser, Fettsäuresalz (Seife) und üblichen kosmetischen Wirk-und Hilfsstoffen als Gelstrukturbildner ein Gemisch aus 0,05 - 4 Gew.-% mindestens eines Celluloseethers und/odermindestens eines Acrylsäurepolymerisats und 10 - 20 Gew.-% mindestens eines Zuckeralkohols, sowie als Nachschäummittel ein Gemisch aus 8 - 20 Gew.-% mindestens eines unter Normalbedingungen flüssigen Fluorchlorkohlenwasserstoffes als Lösungsmittelkomponente und 4 -10 Gew.-% mindestens eines unter Normalbedingungen gasförmigen Fluorchlorkohlenwasserstoffes als Treibgaskomponente enthält.

2. Nachschäumendes kosmetisches Gel gemäß Anspruch 1, dadurch gekennzeichnet, daß es sich um ein nachschäumendes Rasiergel handelt.

3. Nachschäumendes kosmetisches Gel gemäß Anspruch 1, dadurch gekennzeichnet, daß es sich bei dem Zuckeralkohol um einen Polyalkohol mit 3 - 6 C-Atomen in der Kette, vorzugsweise um Sorbit handelt.

4. Nachschäumendes kosmetisches Gel gemäß Anspruch, 1, dadurch gekennzeichnet, daß es sich bei dem als Lösungsmittelkomponente des Nachschäummittels dienenden Fluorchlorkohlenwasserstoff um ein wasserstofffreies Ethan-Derivat handelt, welches nur Fluor-und Chlorsubstituenten trägt.

5. Nachschäumendes kosmetisches Gel gemäß Anspruch 1, dadurch gekennzeichnet, daß es sich bei dem als Lösungsmittelkomponente des Nachschäummittels dienenden Fluorchlorkohlenwasserstoff um 1,1,2-Tricholor-1,2,2-trifluorethan handelt.

6. Nachschäumendes kosmetisches Gel gemäß Anspruch 1, dadurch gekennzeichnet, daß es als Treibgaskomponente des Nachschäummittels ein Gemisch aus einem perhalogenierten, nur Fluor-und Chlorsubstituenten tragenden Methan-und einem ebensolchen Ethan-Derivat enthält.

7. Nachschäumendes kosmetisches Gel gemäß Anspruch 1, dadurch gekennzeichnet, daß es als Treibgaskomponente ein Gemisch aus Dichlordifluormethan und 1,2-Dichlortetrafluorethan in einem Verhältnis von 1 : 2,6 bis 1 : 16 enthält.

8. Nachschäumendes kosmetisches Gel gemäß Anspruch 1, dadurch gekennzeichnet, daß es maximal 60 Gew.-% Wasser enthält.

9. Nachschäumendes kosmetisches Gel gemäß Anspruch 1, dadurch gekennzeichnet, daß es 10-20 Gew.-% eines kosmetisch akzeptablen Salzes einer gesättigten Fettsäure mit 12-18 C-Atomen enthält.

10. Nachschäumendes kosmetisches Gel gemäß Anspruch 1, dadurch gekennzeichnet, daß es sich bei dem Fettsäuresalz um das Triethanolaminsalz der Stearin-, Palmitin-oder Myristinsäure oder Gemische dieser Salze handelt.

11. Verfahren zur Herstellung eines in Aerosoldosen abgefüllten nachschäumenden kosmetischen Gels, dadurch gekennzeichnet, daß eine Zusammensetzung gemäß einem der Ansprüche 1 - 10 in die innere Kammer eines üblichen Zweikammer-Aerosolbehälters abgefüllt wird und die äußere Kammer mit einem komprimierten Gas oder Gasgemisch als Ausbringmittel befüllt wird.